(19) Europäisches Patentamt
European Patent Office
Office européen des brevets

(11) Veröffentlichungsnummer: **0 456 960 A1**

(12) # EUROPÄISCHE PATENTANMELDUNG

(21) Anmeldenummer: **91101398.5**

(51) Int. Cl.5: **C07D 277/06**, C07C 255/26

(22) Anmeldetag: **02.02.91**

Ein Antrag gemäss Regel 88 EPÜ auf Berichtigung der Beschreibung liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: **15.05.90 DE 4015573**

(43) Veröffentlichungstag der Anmeldung:
**21.11.91 Patentblatt 91/47**

(84) Benannte Vertragsstaaten:
**BE CH DE ES FR GB IT LI NL**

(71) Anmelder: **Degussa Aktiengesellschaft**
**Weissfrauenstrasse 9**
**W-6000 Frankfurt am Main 1(DE)**

(72) Erfinder: **Jakob, Harald, Dr.**
**Hauptstrasse 78**
**W-6467 Hasselroth 1(DE)**
Erfinder: **Huthmacher, Klaus, Dr.**
**Lärchenweg 18**
**W-6460 Gelnhausen(DE)**
Erfinder: **Klenk, Hubert, Dr.**
**Gleiwitzer Strasse 21**
**W-6450 Hanau 9(DE)**

(54) **Optisch aktive Salze aus einem substituierten Thiazolidin-4-carboxylat und 3-Cyano-2-hydroxypropyltrimethylammonium, deren Herstellung und Verwendung.**

(57) Die Erfindung betrifft optisch aktive Salze der allgemeinen Formel (I)

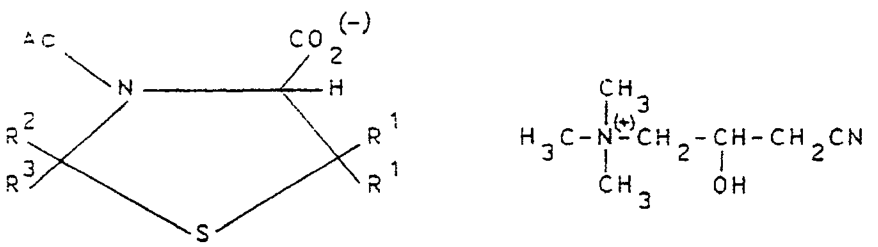

(I),

worin Ac eine Acylgruppe, $R^1$ Wasserstoff oder Methyl und $R^2$ und $R^3$ gleiche oder unterschiedliche Substituenten, vorzugsweise Methyl bedeuten. Die Salze lassen sich aus der entsprechenden optisch aktiven Carbonsäure und DL-3-Cyano-2-hydroxypropyltrimethylammoniumhydroxid gewinnen. Die diastereomeren Salzpaare lassen sich leicht voneinander trennen und wieder spalten, so daß sich das Verfahren zur Herstellung von optisch aktiven 3-Cyano-2-hydroxypropyltrimethylammoniumsalzen eignet.

EP 0 456 960 A1

Die Erfindung betrifft neue optisch aktive Salze aus einem substituierten Thiazolidin-4-carboxylat und 3-Cyano-2-hydroxypropyltrimethylammonium und deren Herstellung und Verwendung. Eine bevorzugte Verwendung ist die Gewinnung von L-(-)-3-Cyano-2-hydroxypropyltrimethylammoniumchlorid (L-Carnitinnitrilchlorid), einem wertvollen Baustein für die Synthese von L-Carnitin.

L-Carnitin, auch als Vitamin $B_T$ bekannt, findet in diätischen und pharamzeutischen Präparaten zunehmend Anwendung zur Behandlung von Herzmuskelschäden, chronischen Durchblutungsstörungen sowie zur Erhöhung der Leistungsfähigkeit. Die meisten chemischen Verfahren zur Herstellung von L-Carnitin schließen eine Racematspaltung einer Carnitin-Vorstufe ein. Bekannt ist eine Racematspaltung der Vorstufe D,L-Carnitinamid unter Verwendung einer optisch aktiven Säure (DD-PS 23217, DE-OS 29 27 672, DE-OS 33 42 713). Nachteilig ist insbesonders, daß D,L-Carnitinamid zunächst aus D,L-Carnitinnitrilchlorid gezielt hergestellt werden muß. Gegenüber einer Racematspaltung auf der Stufe des D,L-Carnitinnitrilchlorids, das direkt zu Carnitin verseift werden kann, ist also eine zusätzliche Stufe erforderlich.

Gemeinsame Merkmale einer Racematspaltung von D,L-Carnitinnitrilchlorid, dessen Herstellung im allgemeinen durch Cyanidierung von D,L-3-Chlor-2-hydroxypropyltrimethylammoniumchlorid oder D,L-Epoxypropyltrimethylammoniumchlorid erfolgt, sind die Überführung des Chlorids in das Hydroxid, Umsetzung mit einer optisch aktiven Säure, Trennung der diastereomeren Salze und Spaltung derselben mit einer starken Säure, wobei das optisch aktive Carnitinnitrilsalz gewonnen und die eingesetzte optisch aktive Säure zurückgewonnen werden.

Als optisch aktive Säure für die Racematspaltung von Carnitinnitrilchlorid wurden z. B. D-Weinsäure und D-Campher-10-sulfonsäure vorgeschlagen, welche aber wegen der geringen Löslichkeitsunterschiede ein häufiges Umkristallisieren der diastereomeren Salze erforderlich machten. Eine Verbesserung brachte zwar die kombinierte Verwendung von D-Champher-10-sulfonsäure und Dibenzoyl-L-weinsäure (E. Strack et al., Z. physiol. Chem 318, 129 (1960)), jedoch verursachen zwei Spaltungsmittel hohe Kosten und machen das Verfahren für eine industrielle Anwendung ungeeignet. Bei alleiniger Verwendung von Dibenzoyl-L-weinsäure ist der Löslichkeitsunterschied zwischen den beiden Diastereomeren gering, was wiederum die Ausbeute beeinträchtigt. Bekannt wurde auch die Verwendung von optisch aktiver N-Acetyl-glutaminsäure als Spaltsäure (JP-B 43-8248, NL-A 6614321): für die Gewinnung des L-Carnitinnitrilsalzes ist nicht die natürlich vorkommende N-Acetyl-L-glutaminsäure, sondern deren Antipode N-Acetyl-D-glutaminsäure, welche selbst nicht in ausreichender Menge verfügbar ist bzw. selbst mittels D-Carnitinnitrilchlorid hergestellt werden muß, erforderlich; trennt man aber zunächst das schwer lösliche Salz aus D-Carnitinnitril und N-Acetyl-L-glutaminsäure von dem Diastereomerenpaar ab, erfordert die Gewinnung eines optisch reinen L-Carnitin-N-acetyl-D-glutamats aus der Mutterlauge ein mehrfaches fraktionierendes Kristallisieren.

Um die Effizienz der vorgenannten Spaltreagenzien zu verbessern und die optische Reinheit des gewünschten Carnitinnitrilsalzes zu erhöhen, wurde vorgeschlagen, eine weitere fraktionierende Kristallisation eines Salzes aus dem zunächst erhaltenen optisch inaktiven Carnitinnitrilhydroxid und einer optisch aktiven Säure, wie Oxalsäure oder Perchlorsäure, nachzuschalten (JP-A 62-286959); hierdurch wurde aber der gesamte Verfahrensaufwand erheblich erhöht. Die Racematspaltung allein durch fraktionierende Kristallisation von D,L-Carnitinnitriloxalat bzw. -perchlorat führt nur zu geringen chemischen und optischen Ausbeuten (FR-A 2 529 545, FR-A 2 536 391).

Bekannt wurden auch Verfahren zur Racematspaltung von D,L-3-Chlor-2-hydroxypropyltrimethylammoniumchlorid, also der Vorstufe zu D,L-3-Cyano-2-hydroxypropyltrimethylammoniumchlorid. Gemäß der EP-A 312 726 wurde Weinsäure als Spaltreagenz eingesetzt. Die Weinsäure läßt jedoch als Spaltreagenz für technische Zwecke zu wünschen übrig, da sie sich wegen ihrer Wasserlöslichkeit aus den wäßrigen Lösungen, die man bei der Freisetzung des L- bzw. D-3-Chlor-2-hydroxypropyltrimethylammoniumchlorids aus dem diastereomeren Salz erhält, nur mit großem Aufwand zurückgewinnen läßt und zudem die Ausbeute, unter anderem wegen irreversibler Nebenreaktionen, nicht befriedigend ist.

Aus der EP-A 312 726 sind optisch aktive Salze aus einem substituierten Thiazolidin-4-carboxylat und 3-Chlor-2-hydroxypropyltrimethylammonium bekannt. Diese Salze werden hergestellt aus einer substituierten optisch aktiven Thiazolidin-4-carbonsäure, das zunächst mittels Trimethylamin in das Trimethylammoniumsalz überführt und anschließend mit Epichlorhydrin umgesetzt wird. Die resultierenden diastereomeren Salzpaare weisen stark unterschiedliche Löslichkeiten auf, so daß sie durch fraktionierende Kristallisation getrennt werden können. Durch Säurespaltung der Salzpaare kann L-(-)-3-Chlor-2-hydroxytrimethylammoniumchlorid gewonnen werden, das in die 3-Cyanoverbindung und durch Hydrolyse in L-(-)-Carnitin überführt werden kann.

Obgleich dieses Verfahren den Vorteil aufweist, daß das bei der Umsetzung neben dem erwünschten L-(-)-3-Chlor-2-hydroxypropylammoniumchlorid anfallende unerwünschte D-Enantiomere ebenso wie die racemische Form für Kationisierungszwecke verwendbar ist, hat es in der Praxis doch einige Nachteile: Die Umsetzung mit Trimethylamin und Epichlorhydrin erfordert wegen der damit verbundenen Geruchsbelästigung und der hohen Toxizität einen außerordentlich hohen technischen Aufwand, weil auch Spuren nicht umgesetzter Rohstoffe aus dem System abgetrennt und einer sicheren Entsorgung zugeführt werden müssen. Der Aufwand für die erforderlichen technischen Maßnahmen, beispielsweise eine Wasserdampfdestillation, einschließlich der für den Umgang mit Epichlorhydrin erforderlichen Sicherheitsvorkehrungen übersteigt rasch den Nutzen, das D-Enantiomere verwerten zu können.

Aufgabe der vorliegenden Erfindung ist es, die Nachteile der bekannten Verfahren zur Herstellung des für die Carnitinsynthese erforderlichen optisch aktiven 3-Cyano-2-hydroxy-trimethylammoniumchlorids zu vermeiden. Eine weitere Aufgabe ist es, solche optisch aktiven Salze aufzuzeigen, die sich auf einfache und wirtschaftliche Weise gewinnen und praktisch ohne Verlust des optisch aktiven Anions dieser Salze zur Racematspaltung des Kations der Salze, also des D,L-3-Cyano-2-hydroxy-trimethylammoniums, eignen.

Gegenstand der Erfindung sind optisch aktive Salze der allgemeinen Formel (I)

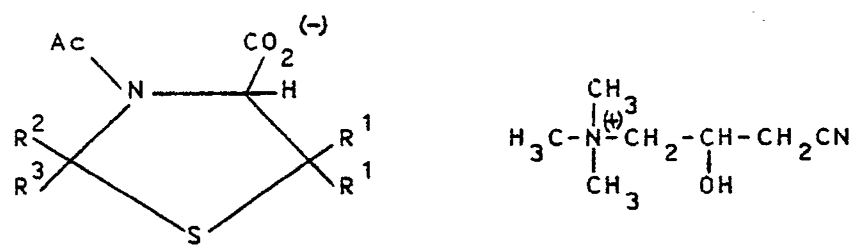

(I),

in der bedeuten:

$R^1$ Wasserstoff oder Methyl;

$R^2$ und $R^3$, welche gleich oder verschieden sein können, Wasserstoff, Alkyl mit 1 bis 8 C-Atomen, Alkenyl mit 2 bis 8 C-Atomen, $C_5$- oder $C_6$-Cycloalkyl oder Aryl oder

$R^2$ und $R^3$ gemeinsam Alkylen mit 4 bis 11 C-Atomen;

Ac eine Acylgruppe.

Besonders bevorzugte Salze sind jene aus D-(+)-3-Formyl-2,2,5,5-tetramethylthiazolidin-4-carboxylat und L-(-)-3-Cyano-2-hydroxypropyltrimethylammonium sowie aus L-(-)-3-Formyl-2,2,5,5-tetramethylthiazolidin-4-carboxylat und D-(+)-3-Cyano-2-hydroxypropyltrimethylammonium.

Vorzugsweise sind $R^2$ und $R^3$ gleich und stehen für Methyl oder Ethyl, oder $R^2$ und $R^3$ bilden gemeinsam Pentamethylen. Bei der Acylgruppe handelt es sich beispielsweise um Benzoyl, Tosyl, Nitrophenylsulfonyl, Acetyl oder Formyl.

Die erfindungsgemäßen optisch aktiven Salze der allgemeinen Formel (I) lassen sich dadurch herstellen, daß man D,L-3-Cyano-2-hydroxypropyltrimethylammoniumhydroxid mit einer optisch aktiven Säure der allgemeinen Formel (II)

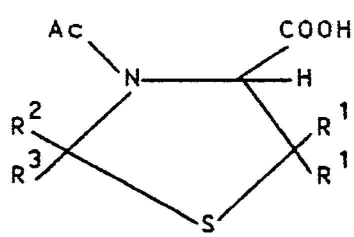

(II),

worin $R^1$, $R^2$, $R^3$ und Ac die Bedeutung gemäß Anspruch 1 haben, umsetzt und, sofern erwünscht, die diastereomeren Salzpaare in an sich bekannter Weise durch fraktionierende Kristallisation voneinander trennt.

Die enantiomerenreinen Thiazolidin-4-carbonsäuren der allgemeinen Formel (II) können in an sich bekannter Weise (vgl. BE-PS 738 520) aus einer optisch aktiven $\beta$-Mercapto-$\alpha$-aminosäure und einem Aldehyd oder Keton mit anschließender Acylierung gewonnen werden; als optisch aktive Säure kann z. B. L-Cystein ($R^1 = H$) oder D- bzw. L-2-Amino-3-methyl-3-mercaptobuttersäure (vgl. GB-PS 585 413, DE-OS 21 38 121) eingesetzt werden.

Bei dem Verfahren der vorliegenden Erfindung wird zunächst D,L-Carnitinnitrilchlorid oder das D,L-Carnitinnitrilsalz einer anderen optisch inaktiven Säure in an sich bekannter Weise in das D,L-Carnitinnitrilhydroxid überführt; hierfür eignen sich besonders Anionenaustauscher oder die Elektrodialyse. Das entstandene D,L-Carnitinnitrilhydroxid wird mit einer äquivalenten Menge einer optisch aktiven Säure der allgemeinen Formel (II) umgesetzt, wobei aus dem optisch aktiven Carboxylat-Gegenion und dem D,L-Carnitinnitrilkation die diastereomeren Salze der allgemeinen Formel (I) entstehen. Die Umsetzung wird vorzugsweise in Gegenwart eines protischen Lösungsmittels, insbesondere Wasser, durchgeführt. Besonders bevorzugt wird das racemische Carnitinnitrilhydroxid in wäßriger Phase vorgelegt und bis zum Neutralpunkt mit der Thiazolidin-4-carbonsäure versetzt. Noch Entfernen des Wassers kann das diastereomere Salzpaargemisch durch fraktionierende Kristallisation aus einem organischen Lösungsmittel in die beiden Salzpaare getrennt werden.

Ein besonderer Vorteil des erfindungsgemäßen Verfahrens liegt darin, daß die Salze der jeweiligen diastereomeren Salzpaare in organischen Lösungsmitteln beträchtliche Löslichkeitsunterschiede aufweisen und daher eine bequeme Trennung in Kristallisat und Mutterlauge gestatten. Im allgemeinen sind die Salze aus den D-Säuren (II) mit dem L-(-)-Carnitinnitril schwerer oder sogar wesentlich schwerer löslich als die diastereomeren DD-Salze; das gleiche gilt natürlich für das Verhältnis der LD-Salze zu den LL-Salzen, so daß auch eine Isolierung der LL-Salze aus der Mutterlauge mit hoher optischer Reinheit möglich ist.

Als Lösungsmittel für die fraktionierende Kristallisation der diastereomeren Salzpaare werden ($C_1$-$C_6$)-Alkohole, insbesondere ($C_1$-$C_4$)-Alkohole, ($C_3$-$C_7$)-Ketone, wie insbesondere Aceton und Methylisobutylketon (MIBK), cyclische Ether, wie z. B. Tetrahydrofuran und Dioxan, Alkylenglykolether mit 3 bis 7 C-Atomen, insbesondere Ethylenglykolmonomethylether, oder Gemische solcher Lösungsmittel verwendet; bevorzugte Lösungsmittelgemische sind n-Butanol/Aceton-, i-Butanol/Aceton-, Methylisobutylketon/Aceton-, n-Butanol/Tetrahydrofuran- oder MIBK/Tetrahydrofuran-Gemische.

Die fraktionierende Kristallisation erfolgt vorzugsweise in wasserfreien Lösungsmitteln. Nach der Salzbildung wird üblicherweise vorhandenes Wasser zunächst abdestilliert, wobei die Mitverwendung Azeotrope mit Wasser bildender Lösungsmittel, insbesondere solcher, welche für die Kristallisation Verwendung finden, vorteilhaft ist. Das schwerer lösliche Salzpaar kristallisiert aus der organischen Lösung beim Abkühlen derselben, z. T. sogar bereits beim Entfernen des Wassers, aus.

Zur Erhöhung der optischen Reinheit kann man das auskristallisierte und abgetrennte Salzpaar noch waschen, zweckmäßig mit Aceton oder Methylisobutylketon, und/oder, soweit erforderlich, umkristallisieren.

Das auf diese Weise mit hoher optischer Reinheit und hoher Ausbeute anfallende Salzpaar wird wie üblich mit achiralen starken Säuren, insbesondere Mineralsäuren und vorzugsweise Salzsäure, in optisch aktives Carnitinnitrilsalz der achiralen Säure und die eingesetzte optisch aktive Thiazolidin-4-carbonsäure gespalten. Das erfindungsgemäße Verfahren bietet den verfahrenstechnisch besonderen Vorteil, daß die Spaltsäuren gemäß der allgemeinen Formel (II) keine irreversiblen Nebenreaktionen eingehen und schwer wasserlöslich sind, so daß sie in einfacher Weise nur mit geringem Extraktions- und Destillationsaufwand in Ausbeuten über 95 % zurückgewonnen werden können. Das Verfahren zur Racematspaltung zeichnet sich ferner durch eine hohe Raum-Zeit-Ausbeute aus.

Durch die hohe optische und gute chemische Reinheit der auskristallisierenden DL- oder LD-Salzpaare stellt auch die Verwendung der L-Säuren zur Kristallisation und Abtrennung des "falschen" LD-Salzpaares ein vorteilhaftes Verfahren dar. Die Isolierung des gewünschten L-Carnitinnitrilchlorids erfolgt nach der oben beschriebenen Spaltung der Mutterlauge mit Salzsäure durch Kristallisation aus einem Alkohol/Wasser-Gemisch.

Die Überführung von L-Carnitinnitrilchlorid in L-Carnitin erfolgt in bekannter Weise durch Erhitzen des Chlorids mit konzentrierter Salzsäure und Umsetzung des anfallenden L-Carnitinchlorids mit einem Kationenaustauscher.

**Beispiel 1**

178,7 g (1,0 Mol) D,L-Carnitinnitrilchlorid wurden in 1 l Wasser gelöst und über eine Ionenaustauschersäule (∅ 55 mm, Länge 90 cm) gepumpt, die mit einem stark basischen Harz (Amberlite IRA-410) in der $OH^-$-Form gefüllt war. Die anfallende D,L-Carnitinnitrilhydroxid-Lösung wurde mit 217,3 g (1,0 Mol) D-3-Formyl-2,2,5,5-tetramethylthiazolidin-4-carbonsäure unter Rühren neutralisiert. Unter vermindertem Druck wurde das Wasser abdestilliert. Der noch warme Rückstand wurde mit 200 ml Methylisobutylketon (MIBK) verrührt und zur restlichen Entwässerung über einen Wasserabscheider andestilliert. Nachdem kein Wasser mehr überdestillierte, versetzte man den ölig-kristallinen Brei mit 400 ml Aceton und rührte kräftig 30 Minuten unter Rückfluß. Die Suspension ließ man auf 20 °C abkühlen, saugte den Niederschlag ab und wusch mit Aceton nach. Das Aceton-feuchte Salzpaar wurde zur Nachreinigung in 300 ml Aceton suspendiert, 30 Minuten bei Siedetemperatur und anschließend noch 2 h bei 20 °C gerührt. Der Niederschlag wurde abgesaugt, mit Aceton gewaschen und getrocknet: 128,7 g (71,5 %) farbloses Salzpaar aus der

eingesetzten D-Thiazolidin-4-carbonsäure und dem L-Carnitinnitrilhydroxid (DL-Salzpaar) mit $(\alpha)_D^{20}$ = +31,7° (c = 1 $H_2O$) Smp.: 158 - 160 °C.

| | | | | |
|---|---|---|---|---|
| Elementaranalyse ber. für $C_{16}H_{29}N_3O_4S$ | C 53,46 | H 8,13 | N 11,69 | S 8,92 |
| gef. | C 53,20 | H 8,40 | N 11,51 | S 8,63 |

**Beispiel 2**

Zur Salzpaar-Spaltung löste man das Salzpaar aus Beispiel 1 in 250 ml Wasser unter Rühren auf, kühlte die Lösung auf 10 °C ab und stellte unter Beibehaltung dieser Temperatur durch Zutropfen von 34,8 g konz. Salzsäure (0,353 Mol) einen pH-Wert von 2 ein. Nach Zugabeende rührte man noch ca. 30 Minuten, saugte den ausgefallenen Niederschlag ab, wusch mit kaltem Wasser nach und trocknete: 72,6 g (93.3 %) farblose D-(+)-3-Formyl-2,2,5,5-tetramethylthiazolidin-4-carbonsäure mit $(\alpha)_D^{20}$ = 52,0° (c = 1, Ethanol).

Das wäßrige Filtrat wurde 3x mit je 40 ml MIBK extrahiert und die vereinigten Extrakte bis zur Trockene einrotiert und getrocknet: 2,7 g (3,5 %) D-Spaltsäure (Rückgewinnung = 96,8 %).

Das extrahierte wäßrige Filtrat wurde zur Trockene einrotiert, mit Aceton digeriert, abgesaugt und getrocknet: 62,7 g (98 %) farbloses L-Carnitinnitrilchlorid mit $(\alpha)_D^{20}$ = -25,9° (c = 1, Wasser).

62,5 g (0,35 Mol) dieses L-Carnitinnitrils wurden unter Rühren in 120 g konzentrierter Salzsäure gelöst und während 6 h stufenweise von 50 auf 80 °C erhitzt, wobei Ammonchlorid ausfiel. Nach Stehenlassen über Nacht wurde die Suspension auf 5 °C abgekühlt, der Niederschlag abfiltriert und mit kalter konz. HCl nachgewaschen. Das Filtat wurde bei max. 70 °C im Vakuum weitgehendst von überschüssiger Salzsäure befreit. Zurück blieb L-Carnitinchlorid als ölig-feststoffhaltiges Gemisch, das zur Freisetzung von L-Carnitin in ca. 300 ml Wasser gelöst und über eine mit 500 ml Amberlite IRA 410/$OH^-$ -Form beladene Ionenaustauschersäule gepumpt wurde. Die anfallende Chlorid-freie Lösung wurde im Vakuum zur Trockene eingeengt. Den leicht gelblichen Rückstand verrührte man mit einem n-Butanol/Aceton-Gemisch. Der entstandene grobkristalline Niederschlag wurde abgesaugt, mit Aceton gewaschen und getrockent: 50,6 g (90 %) farbloses L-Carnitin mit $(\alpha)_D^{20}$ = -31,1° (c = 1, Wasser).

**Beispiel 3**

Eine aus 178.7 g (1 Mol) D,L-Carnitinnitrilchlorid analog Beispiel 1 hergestellte D,L-CAN-hydroxidlösung wurde mit 215,8 g (0,99 Mol) L-3-Formyl-2,2,5,5-tetramethylthiazolidin-4-carbonsäure auf pH = 6,8 gestellt und im Rotationsverdampfer eingeengt. Der noch warme Rückstand wurde mit 200 ml MIBK verrührt und zur Entwässerung unter vermindertem Druck über einen Wasserabscheider andestilliert.

Man erhielt einen ölig-kristallinen Brei, der bei Siedetemperatur 0,5 h mit 400 ml Aceton kräftig verrührt wurde. Nach weiteren 2 h Rühren bei 20 °C wurde der Niederschlag abfiltriert und mit Aceton gewaschen. Das Aceton-feuchte Salzpaar wurde anschließend in 250 ml Aceton suspendiert, für 30 Minuten auf Siedetemperatur erwärmt und 2 h bei 20 °C nachgerührt. Der Niederschlag wurde abgesaugt, mit Aceton gewaschen und getrocknet: 129,2 g (71,8 %) farbloses (LD)-Salzpaar, $(\alpha)_D^{20}$ = -35,2 °C (c = 1, Wasser). Smp.: 159 - 161 °C.

| | | | |
|---|---|---|---|
| Elementaranalyse ber. für $C_{16}H_{29}N_3O_4S$ | C 53,46 | H 8,13 | N 11,69 |
| gef. | C 53,21 | H 8,34 | N 11,75 |

**Beispiel 4**

126 g (0,35 Mol) (LD)-Salzpaar aus Beispiel 3 wurden in 200 ml Wasser gelöst und unter Rühren bei 10-15 °Cmit 34,8 g (0,353 Mol) 37 %iger Salzsäure auf pH = 2,0 gestellt. Die ausgefallene L-3-Formyl-2,2,5,5-thiazolidin-4-carbonsäure wurde abgesaugt, mit Wasser gewaschen und getrocknet: 71,0 g (93,4 %) farblose L-Spaltsäure mit $(\alpha)_D^{20}$ = -52,0° (c = 1, Ethanol).

Das Filtrat wurde 3x mit je 50 ml MIBK extrahiert, die vereinigten Extrakte bis zur Trockene einrotiert und getrocknet: 2,7 g (3,5 %) Spaltsäure (Rückgewinnung = 96.9 %).

Das extrahierte wäßrige Filtrat wurde zur Trockene einrotiert, mit Aceton digeriert und getrocknet: 61.2 g (97,9 %) farbloses D-Carnitinnitrilchlorid mit $(\alpha)_D^{20}$ = +25,8° (c = 1, Wasser).

**Beispiel 5**

Die Mutterlauge der Racematspaltung sowie der (LD)-Salzpaarnachbehandlung aus Beispiel 3 wurden vereinigt und zunächst bei Normaldruck, gegen Ende der Destillation unter vermindertem Druck Aceton abdestilliert. Nach Abkühlen auf 20 °C wurde die gelbe Lösung mit 450 ml Wasser verrührt. Aus dem 2-phasigen Gemisch wurde die gelb gefärbte MIBK-Phase abgetrennt. Die wäßrige Phase wurde zur Entfernung restlichen MIBKs im Teilvakuum angestrippt. Anschließend kühlte man die wäßrige Mutterlauge ab und stellte die Lösung bei 10-15 °C Innentemperatur unter Rühren und Kühlen mit 62 g (0,628 Mol) konz. Salzsäure auf pH = 2,0.

Danach wurde noch 30 min bei 10 °C gerührt und die ausgefallene L-Spaltsäure abgesaugt, mit Wasser gewaschen und getrocknet: 131,0 g (94,1 %) farblose L-3-Formyl-2,2,5,5-tetramethylthiazolidin-4-carbonsäure mit $(\alpha)_D^{20}$ = 51,7° (c = 1, Ethanol).

Das Filtrat wurde 3x mit 50 ml MIBK extrahiert und die vereinigten org. Phasen zur Trockene eingeengt: 3,4 g (2,4 %) L-Spaltsäure (Rückgewinnung = 96,5 %).

Die wäßrige Lösung wurde bis zu einem noch gut rührbaren Kristallbrei eingeengt, mit 150 ml n-Butanol versetzt und unter vermindertem Druck zur vollständigen Entwässerung über einen Wasserabscheider andestilliert. Die erhaltene Suspension wurde bei Siedetemperatur mit 300 ml Methanol mit 10 Vol-% Wasser versetzt. Nach Erkaltenlassen auf RT wurde noch 1 h nachgerührt. Der ausgefallene Niederschlag wurde abgesaugt, mit Methanol gewaschen und getrockent: 62,8 g (35 % bezogen auf eingesetzte D,L-Verbindung) L-Carnitinnitrilchlorid mit $(\alpha)_D^{20}$ = -25,9° (c = 1, Wasser).

60,8 g (0,34 Mol) des so erhaltenen L-Carnitinnitrilchlorids wurden mit 108 g 37 %iger Salzsäure wie in Beispiel 2 verseift und aufgearbeitet: 50,4 g (91,9 %) L-Carnitin mit $(\alpha)_D^{20}$ = -30,8° (c = 1, Wasser), HPLC > 99 %.

**Beispiel 6**

Eine aus 60,7 g D,L-Carnitinnitrilchlorid analog Beispiel 1 hergestellte D,L-Carnitinnitrilhydroxidlösung wurde mit 78,5 g (0,34 Mol) L-(-)-3-Formyl-2,2-pentamethylenthiazolidin-4-carbonsäure auf pH = 6,0 gestellt und im Rotationsverdampfer eingeengt. Der Rückstand wurde mit 610 ml n-Butanol verrührt und zur Entwässerung über einen Wasserabscheider andestilliert. Nach Abkühlen auf 20 °C wurde noch 2 h gerührt und anschließend das auskristallisierte LD-Salzpaar aus der L-Thiazolidin-4-carbonsäure und dem D-Carnitinnitrilhydroxid abfiltriert und getrocknet: Ausbeute 38,6 g (61,1 %).

33,4 g des LD-Salzpaares wurden aus 130 ml n-Butanol/Aceton umkristallisiert und die nach Trocknen erhaltenen 26,3 g wie in Beispiel 2 beschrieben gespalten. Man erhielt ohne Extraktion der wäßrigen Mutterlauge 16,2 g (99 %) L-(-)-3-Formyl-2,2-pentamethylenthiazolidin-4-carbonsäure zurück. Das wäßrige Filtrat wurde eingeengt und getrocknet: 12,3 g (97,2 %) D-Carnitinnitrilchlorid (46,7 % d. Th.) mit $(\alpha)_D^{20}$ = +22,1° (c = 1, Wasser).

Die Mutterlaugen der Racematspaltung sowie der Salzpaarumkristallisation wurden vereinigt und eingedampft. Der verbliebene Feststoff wurde in 280 ml Wasser gelöst und bei 0 °C mit 50 g konz. Salzsäure versetzt. Die ausgefallene L-Thiazolidincarbonsäure wurde abgesaugt und getrocknet: 56,3 g (96,2 %). Das wäßrige Filtrat wurde eingedampft und der kristalline Rückstand aus wäßrigem Methanol umkristallisiert: 27.34 g (90 %) L-Carnitinnitrilchlorid mit $(\alpha)_D^{20}$ = -10,9 °C (c = 1, Wasser). Eine weitere Umkristallisation von 26,5 g lieferte 11,88 g (40,4 % d. Th.) L-Carnitinnitrilchlorid mit $(\alpha)_D^{20}$ = -22,9° (c = 1, Wasser).

**Beispiel 7**

Wie in Beispiel 1 beschrieben, wurden 55,4 kg D,L-Carnitinnitrilchlorid (Gehalt 96,8 %) in 300 l Wasser gelöst und am Ionenaustauscher (400 l Amberlite IRA-416, $OH^-$-Form) in die Hydroxidform überführt. Nach Neutralisation mit 68,3 kg D-3-Formyl-2,2,5,5-tetramethylthiazolidin-4-carbonsäure wurde unter vermindertem Druck Wasser abdestilliert. Restliche Mengen an Wasser entfernte man nach Zugabe von 60 l MIBK durch azeotrope Destillation am Wasserabscheider. Anschließend wurde zur DL-Salzpaar-Kristallisation 120

l Aceton zugegeben und für 0,5 h erhitzt. Nach Abkühlen auf Raumtemperatur wurde das DL-Salzpaar abzentrifugiert, auf der Zentrifuge 3x mit je 30 l Aceton gewaschen und getrocknet: 40,6 kg (75,3 % d. Th.) DL-Salzpaar, das zur Spaltung wie in Beispiel 2 beschrieben in 75 l Wasser gelöst und bei 10 °C mit 97 l konz. Salzsäure versetzt wurde. Die ausgefallene D-Thiazolidincarbonsäure wurde abzentrifugiert, mit Wasser gewaschen und feucht (30,1 kg) in den Spaltprozeß zurückgeführt (Rückgewinnungsquote 94 %). Das wäßrige Filtrat wurde 2x mit 15 l MIBK extrahiert. Aus den vereinigten Extrakten gewann man restliches Spaltreagens (2,5 %) zurück.

Die wäßrige L-Carnitinnitrilchlorid-haltige Mutterlauge (Eindampfung und Trocknung einer Probe ergab $(\alpha)_D^{20}$ = -25,9° ; c = 1, $H_2O$) wurde aufkonzentriert. mit 32,9 l konz. Salzsäure versetzt und wie in Beispiel 2 beschrieben zu L-Carnitinchlorid verseift. Dieses wurde anschließend am Ionenaustauscher (Amberlite IRA-416) zum Betain umgesetzt. Man isolierte 15,9 kg farbloses L-Carnitin (32 % bezogen auf D,L-Carnitinnitrilchlorid) mit $(\alpha)_D^{20}$ = -30,7° und einem HPLC-Gehalt > 99 %.

**Beispiel 8**

Wie in Beispiel 1 beschrieben, wurden 56,3 kg D,L-Carnitinnitrilchlorid (Gehalt 95,0%) in 300 l Wasser gelöst und am Ionenaustauscher (Amberlite IRA-416, $OH^-$-Form) in die Hydroxidform überführt. Nach Neutralisation mit 68,0 kg L-3-Formyl-2,2,5,5-tetramethylthiazolidin-4-carbonsäure wurde unter vermindertem Druck Wasser abdestilliert. Restliche Mengen an Wasser entfernte man nach Zugabe von 60 l MIBK durch azeotrope Destillation am Wassserabscheider. Anschließend wurde zur LD-Salzpaar-Kristallisation 120 l Aceton zugegeben und für 0,5 h erhitzt. Nach Abkühlen auf Raumtemp. wurde das LD-Salzpaar abzentrifugiert, auf der Zentrifuge 3x mit je 30 l Aceton gewaschen und getrocknet: 37,0kg (69,4% d. Th.) LD-Salzpaar, das zur Spaltung wie in Beispiel 4 beschrieben in 75 l Wasser gelöst und bei 10 °C mit 8,2 l konz. Salzsäure versetzt wurde. Die ausgefallene L-Thiazolidincarbonsäure wurde abzentrifugiert, mit Wasser gewaschen und feucht (25,9 kg) in den Spaltprozeß zurückgeführt.

Wie in Beispiel 5 beschrieben wurden die Mutterlaugen der Racematspaltung sowie der Aceton-Wäsche vereinigt und das Aceton destillativ abgetrennt. Anschließend versetzte man mit 130 l Wasser und trennte die Phasen. Die wässrige Phase wurde nach A-Kohle-Behandlung gestrippt, auf 10 °C gekühlt und mit 15,9 l konz. Salzsäure auf pH 2 gestellt. Die ausgefallene L-Thiazolidincarbonsäure wurde abzentrifugiert, mit Wasser gewaschen und feucht (59,5 kg) in den Spaltprozeß zurückgeführt.

Die Mutterlauge wurde nach 3-maliger Extraktion mit MIBK (Zurückgewinnung restlicher Spaltsäure) weitgehendst eingedampft, mit 90 l Methanol versetzt und 30 min unter Rückfluß erhitzt. Nach Abkühlen lassen auf Raumtemp. wurden 27,7 kg feuchtes L-Carnitinnitrilchlorid ($[a]_D^{20}$ = -22,9°) isoliert, das man zur weiteren Reinigung in 40 l Methanol 30 min erhitzte. Nach Filtration, Waschen mit 20 l Methanol und Trocknung erhielt man 18,2kg (34% bezogen auf D,L-Carnitinnitrilchlorid) mit $[\alpha]_D^{20}$ = -25,6° (c = 1,Wasser); HPLC-Gehalt: >99,9%. Die Mutterlaugen der L-Carntinnitrilchlorid-Isolierung wurden vereinigt und bis zur Kristallbildung eingedampft. Nach Abkühlen auf Raumtemp. wurden 10,8kg D,L-Carnitinnitrilchlorid $[\alpha]_D^{20}$ = -3,0°) isoliert und in den Spaltprozeß zurückgeführt. 13,5 kg L-Carnitinnitrilchlorid wurden in 22 l konz. Salzsäure gelöst und wie in Beispiel 2 beschrieben zu L-Carnitinchlorid hydrolisiert. Dieses wurde anschließend am stark basischen Ionenaustaustauscher (Amberlite IRA-416) zum Betain umgesetzt. Nach entsprechender Aufarbeitung isolierte man 11,1 kg farbloses L-Carnitin mit $[\alpha]_D^{20}$ = -30.80° und einem HPLC-Gehalt von 99,7%.

**Patentansprüche**

1. Optisch aktive Salze der allgemeinen Formel (I)

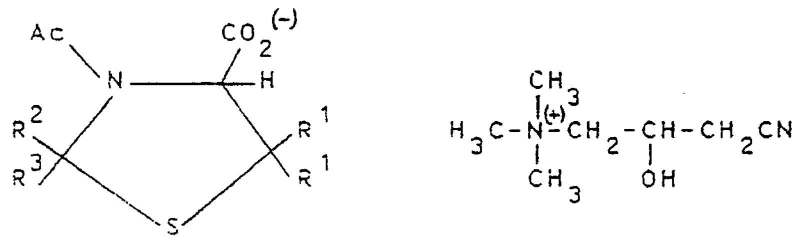

(I),

in der bedeuten:

$R^1$ Wasserstoff oder Methyl;

$R^2$ und $R^3$, welche gleich oder verschieden sein können, Wasserstoff, Alkyl mit 1 bis 8 C-Atomen, Alkenyl mit 2 bis 8 C-Atomen, $C_5$- oder
$C_6$-Cycloalkyl oder Aryl oder $R^2$ und $R^3$ gemeinsam Alkylen mit 4 bis 11 C-Atomen;

Ac eine Acylgruppe.

2. Salz aus D-(+)-3-Formyl-2,2,5,5-tetramethylthiazolidin-4-carboylat und L-(-)-3-Cyano-2-hydroxypropyltrimethylammonium.

3. Salz aus L-(-)-3-Formyl-2,2,5,5-tetramethylthiazolidin-4-carboxylat und D-(+)-3-Cyano-2-hydroxypropyltrimethylammonium.

4. Verfahren zur Herstellung eines optisch aktiven Salzes gemäß Anspruch 1,
dadurch gekennzeichnet,
daß man D,L-3-Cyano-2-hydroxypropyltrimethylammoniumhydroxid mit einer optisch aktiven Säure der allgemeinen Formel (II)

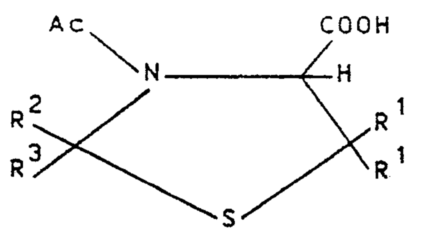

(II),

worin $R^1$, $R^2$, $R^3$ und Ac die Bedeutung gemäß Anspruch 1 haben, umsetzt und, sofern erwünscht, die diastereomeren Salzpaare in an sich bekannter Weise durch fraktionierende Kristallisation voneinander trennt.

5. Verfahren nach Anspruch 4,
dadurch gekennzeichnet,
daß man die Umsetzung in Gegenwart von Wasser und die fraktionierende Kristallisation in einem vorzugsweise wasserfreien organischen Lösungsmittel aus der Reihe ($C_1$-$C_6$)-Alkohole, ($C_3$-$C_7$)-Ketone, cyclische Ether, Alkylenglykolether mit 3 bis 7 C-Atomen oder Gemischen solcher Lösungsmittel durchführt.

6. Verwendung der optisch aktiven Salze nach den Ansprüchen 1 bis 3 zur Herstellung von optisch aktiven 3-Cyano-2-hydroxypropyltrimethylammoniumsalzen, wobei das Anion achiral und vorzugsweise Chlorid ist.

Europäisches Patentamt

# EUROPÄISCHER RECHERCHENBERICHT

Nummer der Anmeldung

**EP 91 10 1398**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 312 726 (DEGUSSA)<br>* Insgesamt * | 1-6 | C 07 D 277/06<br>C 07 C 255/26 |
| Y | FR-A-2 536 391 (SANOFI)<br>* Patentansprüche; Seiten 1-3 * | 1-6 | |
| Y | DE-A-1 907 035 (TANABE SEIYAKU)<br>* Patentansprüche; Seite 6 * | 1-6 | |
| Y | ER-A-1 573 15 (LONZA AG)<br>* Patentansprüche; Seiten 6,7; Seiten 12,13, Beispiel 6 * | 1-6 | |

RECHERCHIERTE SACHGEBIETE (Int. Cl.5)

C 07 D 277/00
C 07 C 255/00

Der vorliegende Recherchenbericht wurde für alle Patentansprüche erstellt

| Recherchenort | Abschlußdatum der Recherche | Prüfer |
|---|---|---|
| Den Haag | 31 Juli 91 | HENRY J.C. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument